# EUROPÄISCHE PATENTSCHRIFT

(11) **EP 4 558 068 B1**
(45) Veröffentlichungstag und Bekanntmachung des Hinweises auf die Patenterteilung: **02.09.2026**
(21) Anmeldenummer: 23744669.5
(22) Anmeldetag: 11.07.2023
(51) Int. Cl.: A61B 17/29

(54) **CHIRURGISCHES INSTRUMENT**
SURGICAL INSTRUMENT
INSTRUMENT CHIRURGICAL

(30) Priorität: 18.07.2022 DE 102022207325
(43) Veröffentlichungstag der Anmeldung: 28.05.2025
(73) Patentinhaber: Richard Wolf GmbH, 75438 Knittlingen (DE)
(72) Erfinder: KÖRNER, Eberhard, 75438 Knittlingen (DE); MAUL, Silas, 75180 Pforzheim (DE); FRATTINI, Vincent, 76137 Karlsruhe (DE)
(74) Vertreter: Patentanwälte Hemmer Lindfeld Frese Partnerschaft mbB
(86) Internationale Anmeldenummer: PCT/DE2023/200141
(87) Internationale Veröffentlichungsnummer: WO 2024/017446

(56) Entgegenhaltungen:
- DE-A1- 10 236 274
- DE-B3- 10 328 514
- DE-B4- 10 110 106

## Beschreibung

Die vorliegende Offenbarung betrifft chirurgische Instrumente zum chirurgischen Greifen, Kneifen, und/oder Schneiden von Gewebe innerhalb eines organischen Körpers, insbesondere laparoskopische Instrumente.

Chirurgische Instrumente für endoskopische Operationen, wie etwa laparoskopische Instrumente, sind häufig möglichst dünn ausgestaltet, um den chirurgischen Eingriff für den Patienten möglichst minimalinvasiv durchführen zu können. Der Werkzeugkopf eines solchen chirurgischen Instruments weist daher typischerweise relativ filigrane Werkzeugteile wie etwa Maulteile einer (Kneif-)Zange oder Klingen einer Schere auf. Es besteht daher grundsätzlich ein Risiko, dass die auf den Werkzeugkopf übertragende manuelle Kraft der Anwenderperson so groß ist, dass filigrane Werkzeugteile brechen.

Es ist daher bekannt, dass solche manuell bedienbaren chirurgische Instrumente eine Kraftbegrenzungsvorrichtung oder Überlastungsschutzeinrichtung aufweisen, welche das Risiko eines Bruchs der Werkzeugteile erheblich reduziert.

Beispielsweise sind solche Überlastungsschutzeinrichtungen aus der DE 101 10 106 B4, der DE 102 36 274 A1, der DE 103 28 514 B3, der DE 299 17 554 U1, der US 5,009,661 A, der DE 44 11 099 C2, oder der DE 297 13 490 U1 bekannt.

Die bekannten Überlastungsschutzeinrichtungen haben allerdings den Nachteil, dass die auf den Werkzeugkopf übertragende manuelle Kraft bei kleinen Öffnungswinkeln des Werkzeugs relativ gering ist. Daher können in bestimmten Situationen kleine Gewebestücke nicht mit der notwendigen Kraft gegriffen, abgekniffen und/oder zerschnitten werden. Insbesondere bei einem häufig benötigten Pinzettengriff mit dem äußersten distalen Ende des Werkzeugkopfs ist die Greif-, Kneif oder Schneidkraft besonders gering.

Der Grund dafür liegt darin, dass die Federkennlinie einer üblicherweise als Überlastungsschutzeinrichtung dienenden Feder eine Ursprungsgerade ist und die Feder am Anfang der Federauslenkung nur geringe Federkraft aufweist. Reicht bei kleinen Öffnungswinkeln des Werkzeugs der weitere Bewegungsspielraum der Betätigungshebel nicht aus, um die Federkraft zu erhöhen, bleibt die auf den Werkzeugkopf übertragende manuelle Kraft relativ gering. Ein kleines Gewebestück könnte daher aus dem Pinzettengriff entgleiten oder nicht erfolgreich abgekniffen und/oder zerschnitten werden.

Es ist daher die Aufgabe der vorliegenden Erfindung, den Werkzeugkopf des chirurgischen Instruments einerseits vor Überlastung zu schützen und andererseits eine ausreichend hohe Kraftübertragung bei kleinen Öffnungswinkeln des Werkzeugs bereitzustellen.

Gelöst wird diese Aufgabe durch den Gegenstand des unabhängigen Anspruchs 1. Bevorzugte Ausführungsformen können den Unteransprüchen, der Beschreibung sowie den Figuren entnommen werden.

Gemäß der vorliegenden Offenbarung wird ein chirurgisches Instrument bereitgestellt zum chirurgischen Greifen, Kneifen, und/oder Schneiden von Gewebe innerhalb eines organischen Körpers, wobei das chirurgische Instrument aufweist:
- eine Betätigungseinrichtung mit zwei Griffteilen, wobei mindestens ein erstes der zwei Griffteile relativ zu einem zweiten der zwei Griffteile translatorisch und/oder rotatorisch in eine Öffnungs- und Schließrichtung beweglich gelagert ist,
- einen Werkzeugkopf mit zwei Werkzeugteilen, wobei mindestens ein erstes der zwei Werkzeugteile relativ zu einem zweiten der zwei Werkzeugteile translatorisch und/oder rotatorisch in eine Öffnungs- und Schließrichtung beweglich gelagert ist,
- einen sich zwischen der Betätigungseinrichtung und dem Werkzeugkopf entlang einer Längsachse erstreckenden Schaft, wobei ein proximales Ende des Schafts mit der Betätigungseinrichtung verbunden oder verbindbar ist, und wobei ein distales Ende des Schafts mit dem Werkzeugkopf verbunden oder verbindbar ist, wobei der Schaft ein axial in einer Öffnungs- und Schließrichtung bewegliches Übertragungselement zur Übertragung einer manuellen Kraft von dem ersten Griffteil auf das erste Werkzeugteil aufweist, und
- eine Überlastungsschutzeinrichtung mit einem Federelement zur Begrenzung der in Schließrichtung auf das erste Werkzeugteil übertragenen manuellen Kraft.
Das chirurgische Instrument ist dadurch gekennzeichnet, dass die Überlastungsschutzeinrichtung einen Kniehebelmechanismus aufweist, über welchen das Federelement das Übertragungselement in Schließrichtung vorspannt.

Durch die Vorspannung wird sichergestellt, dass gleich zu Beginn der Auslenkung des Federelements eine ausreichend hohe Kraftübertragung auf das Werkzeug erzielt wird, unabhängig vom Öffnungswinkel des Werkzeugkopfs. Das bedeutet, dass bei kleinen Öffnungswinkeln des Werkzeugkopfs mindestens die Vorspannkraft des Federelements auf das Werkzeug übertragen wird. Durch entsprechende Ausgestaltung der Parameter des Kniehebelmechanismus und des Federelements kann eine gewünschte Vorspannkraft gewährleistet werden, damit selbst kleinste Gewebestücke erfolgreich im Pinzettengriff gekniffen und/oder zerschnitten werden können bzw. nicht aus einem Zangengriff entgleiten.

Ein weiterer Vorteil des Kniehebelmechanismus ist es, dass die Kraftübertragung auf das Werkzeug auf dem Federweg sogar reduziert werden kann bei größeren Gewebestücken, die einen größeren Öffnungswinkel des Werkzeugkopfs benötigen. Für größere Gewebestücke wird der Pinzettengriff mit dem distalen Ende des Werkzeugkopfs nämlich gar nicht benötigt, und im genutzten mittleren oder proximalen Bereich der Werkzeugteile aufgrund der Hebelwirkung prinzipiell weniger Kraft benötigt. Zudem spielen in der Praxis große, harte Gewebestücke kaum eine Rolle.

Optional kann der Kniehebelmechanismus in einem Kniepunkt mit dem Übertragungselement gelenkig verbunden sein. Dies ist vorteilhaft, um bei möglichst unkompliziertem Aufbau des Kniehebelmechanismus und minimaler Teilevielfalt die Kraft aus dem Federelement in Richtung der Längsachse auf das Übertragungselement einzuleiten.

Erfindungsgemäß weist der Kniehebelmechanismus zwei radial von der Längsachse beabstandete distale Lagerpunkte auf, über welche der Kniehebelmechanismus jeweils mit einem von zwei Federbügeln des Federelements gelenkig verbunden ist. Damit kann ein zur Längsachse im Wesentlichen symmetrischer Aufbau der Überlastungsschutzeinrichtung erzielt werden, wobei die Federkraft gleichmäßig auf die zwei Federbügel verteilt wird. Die Federbügel können Teil eines einstückigen Federelements sein, wobei sich die Federbügel unter Verbiegung des Federelements federnd radial nach außen schwenken. Das Federelement kann also zwei federnde proximale Festkörpergelenke ausbilden, von denen sich jeweils die Federbügel zu dem jeweiligen distalen Lagerpunkt erstrecken.

Optional kann der Kniepunkt proximal von den distalen Lagerpunkten angeordnet sein. Dadurch definiert der Kniehebelmechanismus im Kniepunkt einen distalwärts geöffneten Öffnungswinkel. Werden die distalen Lagerpunkte in Schließrichtung proximalwärts gezogen und der Kniepunkt verspürt einen durch ein Gewebestück im Werkzeug bedingten Widerstand gegen eine weitere Bewegung in Schließrichtung, so vergrößert sich der Öffnungswinkel des Kniehebelmechanismus gegen die vorgespannte Federkraft des Federelements.

Optional kann das Federelement einen proximalen Lagerpunkt aufweisen, über welchen das Federelement mit dem ersten Griffteil gelenkig verbunden ist. Dieser proximale Lagerpunkt liegt vorzugsweise auf der Längsachse des Schafts und bewegt sich proximalwärts in Schießrichtung und distalwärts in Öffnungsrichtung. Am proximalen Lagerpunkt zieht das erste Griffteil das Federelement vorzugsweise proximalwärts, wenn das erste Griffteil in Schließrichtung bewegt wird.

Optional kann der proximale Lagerpunkt proximal vom Kniepunkt angeordnet sein. Am Kniepunkt zieht damit das Federelement über den Kniehebelmechanismus das Übertragungselement proximalwärts in Schließrichtung. Bei proximalwärtiger Bewegung des Übertragungselements schließen sich dann die Werkzeugteile des Werkzeugkopfs.

Optional kann das Federelement derart mit dem ersten Griffteil gekoppelt sein, dass es sich gegen die eigene Federspannung bei Bewegung des ersten Griffteils in Schließrichtung unter Öffnung des Kniehebelmechanismus radial nach außen aufspreizt, sobald die weitere Bewegung des ersten Werkzeugteils in Schließrichtung durch Gewebe blockiert wird. Vorzugsweise ist der Reibungswiderstand zur Bewegung des Übertragungselements und der Werkzeugteile so gering, dass sich ohne Blockade der Werkzeugteile durch Gewebe keine Aufspreizung des Federelements ergibt.

Optional kann das Federelement zwei Federbügel aufweisen, die sich auf gegenüberliegenden Seiten lateral von der Längsachse vom ersten Griffteil zum Kniehebelmechanismus erstrecken. Damit kann eine zur Längsachse im Wesentlichen symmetrischer Ausgestaltung der Überlastungsschutzeinrichtung erzielt werden, wobei die Federkraft gleichmäßig auf die zwei Federbügel verteilt wird.

Optional kann das Federelement eine sich entlang der Längsachse erstreckende Führungsöffnung aufweisen, in welcher der Kniepunkt geführt axial beweglich gelagert ist. Durch die Länge und Position der Führungsöffnung kann der Öffnungswinkelbereich des Kniehebelmechanismus und somit der Auslenkungsbereich des Federelements definiert werden. Befindet sich der Kniepunkt beispielsweise am proximalen Ende der Führungsöffnung, so ist der Öffnungswinkel des Kniehebelmechanismus und somit die Federauslenkung minimal. Befindet sich der Kniepunkt beispielsweise am distalen Ende der Führungsöffnung, so ist der Öffnungswinkel des Kniehebelmechanismus und somit die Federauslenkung maximal. Die Führungsöffnung ist vorzugsweise ein sich entlang der Längsachse erstreckendes Langloch im Federelement. Eine Überspannung des Federelements in einen nicht-elastischen Bereich ist damit ausgeschlossen, da der Kniepunkt am distalen Ende der Führungsöffnung anschlägt. Durch geeignete Position des proximalen Endes der Führungsöffnung und der Länge der Kniehebelbeine des Kniehebelmechanismus kann das Federelement vorgespannt werden. Das bedeutet, dass es der Überwindung der Vorspannkraft bedarf, um den Kniepunkt distalwärts vom proximalen Ende der Führungsöffnung weg zu bewegen.

Optional kann das Federelement mit einer Vorspannkraft vorgespannt sein, wenn der Kniehebelmechanismus in einem minimalen Öffnungswinkel steht, wobei sich der Öffnungswinkel des Kniehebelmechanismus gegen die Vorspannkraft weitet, sobald die weitere Bewegung des ersten Werkzeugteils in Schließrichtung durch Gewebe blockiert wird. Vorzugsweise kann dabei die in Schließrichtung auf das erste Werkzeugteil übertragene manuelle Kraft in einem ersten Öffnungswinkelbereich des Kniehebelmechanismus höher sein als die Vorspannkraft und in einem oberhalb des ersten Öffnungswinkelbereichs liegenden zweiten Öffnungswinkelbereich des Kniehebelmechanismus niedriger sein als die Vorspannkraft. Bei großen Gewebestücken wird nämlich kein Pinzettengriff und somit weniger Kraft für das Werkzeug benötigt. Es kann ein über den gesamten Öffnungswinkelbereich des Kniehebelmechanismus bzw. über den gesamten Federweg bzw. über den gesamten Weg des Kniepunkts in der Führungsöffnung eine sich nur in einem relativ geringen Rahmen ändernde Kraft auf das Übertragungselement übertragen werden.

Optional kann der Kniehebelmechanismus zwei gleich lange im Kniepunkt miteinander gelenkig verbundene Kniehebelbeine aufweisen, wobei die Länge der Kniehebelbeine größer ist als ein axialer Bewegungsspielraum des Kniepunkts relativ zu einem proximalen Lagerpunkt des Federelements, über welchen das Federelement mit dem ersten Griffelement gelenkig verbunden ist. Alternativ dazu kann der Kniehebelmechanismus nur ein Kniehebelbein aufweisen, sofern der Kniepunkt in Richtung der Längsachse geführt gelagert ist. Zwei symmetrisch zur Längsachse angeordnete Kniehebelbeine haben allerdings den Vorteil, dass sich Querkräfte im Wesentlichen aufheben und nicht von einer Führung aufgenommen werden müssen.

Der axiale Bewegungsspielraum des Kniepunkts relativ zu einem proximalen Lagerpunkt des Federelements ist vorzugsweise durch die Länge der Führungsöffnung im Federelement bestimmt. Je größer die Länge der Kniehebelbeine ist, umso flacher ist der Verlauf der Kurve der auf das Übertragungselement übertragenen Kraft in Abhängigkeit der Position des Kniepunkts in der Führungsöffnung. Eine solche flache Kraftkurve ist erwünscht, um eine möglichst konstante auf das Übertragungselement übertragene Kraft zu erzielen. Eine flache Kraftkurve kann alternativ oder zusätzlich durch eine niedrige Federkonstante des Federelements erreicht werden, wobei allerdings diese ein bestimmtes Mindestmaß für die gewünschte Vorspannung nicht unterschreiten darf. Es ist ebenfalls für eine flache Kraftkurve günstig, wenn der minimale Öffnungswinkel des Kniehebelmechanismus möglichst groß ist, also wenn der Kniepunkt am proximalen Ende der Führungsöffnung steht, wobei auch hier die gewünschte Vorspannung ein oberes Limit setzt.

Optional kann der Werkzeugkopf eine Zange, eine Schere und/oder eine Kneifzange bilden.

Optional kann das zweite Griffelement starr mit dem Schaft verbunden oder verbindbar sein.

Optional kann das erste Griffelement in einer Schließstellung anschlagen, wenn das erste Werkzeugteil ohne blockierendes Gewebe in einer Schließstellung bereits angeschlagen hat. Ohne blockierendes Gewebe kann dann der Werkzeugkopf nicht überlastet werden. Außerdem kann sich dadurch bei kleinen zu fassenden, kneifenden oder schneidenden Gewebestücken ein effektiv viel kürzerer axialer Bewegungsspielraum des Kniepunkts in der Führungsöffnung ergeben als dessen Länge prinzipiell erlaubt. Insofern ist für kleine Gewebestücke nur ein kurzes Stück der Kraftkurve relevant, das mit der Vorspannkraft beginnt und dann möglichst flach verläuft.

Die vorliegende Offenbarung wird im Folgenden anhand der beiliegenden Figuren genauer erläutert. Dabei zeigen
- Fig. 1a,b: Seitenansichten auf ein Ausführungsbeispiel eines hierin offenbarten chirurgisches Instruments nah an einer Schließstellung (Fig. 1a) und in einer weit geöffneten Stellung (Fig. 1b);
- Fig. 2: eine detaillierte Seitenansicht auf einen proximalen Teil des in Fig. 1a,b gezeigten chirurgischen Instruments;
- Fig. 3: eine detaillierte Seitenansicht auf das Federelement des in Fig. 1a,b gezeigten chirurgischen Instruments;
- Fig. 4: eine detaillierte Seitenansicht auf die Überlastungsschutzeinrichtung des in Fig. 1a,b gezeigten chirurgischen Instruments;
- Fig. 5: eine Darstellung der Kraftwirkungen in der Überlastungsschutzeinrichtung des in Fig. 1a,b gezeigten chirurgischen Instruments; und
- Fig. 6a,b: eine Darstellung bestimmter Parametergrößen der Überlastungsschutzeinrichtung des in Fig. 1a,b gezeigten chirurgischen Instruments sowie ein Kraftkurvendiagramm.

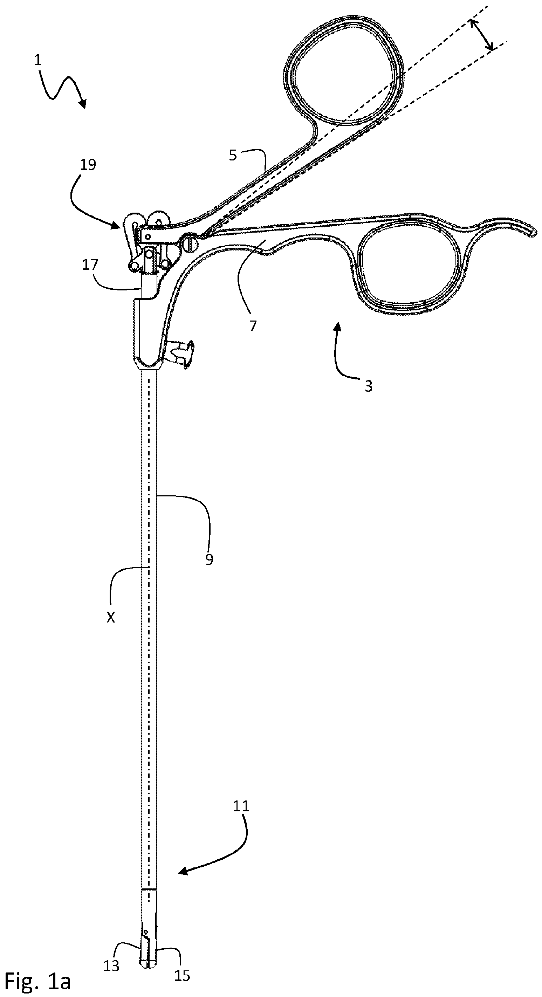

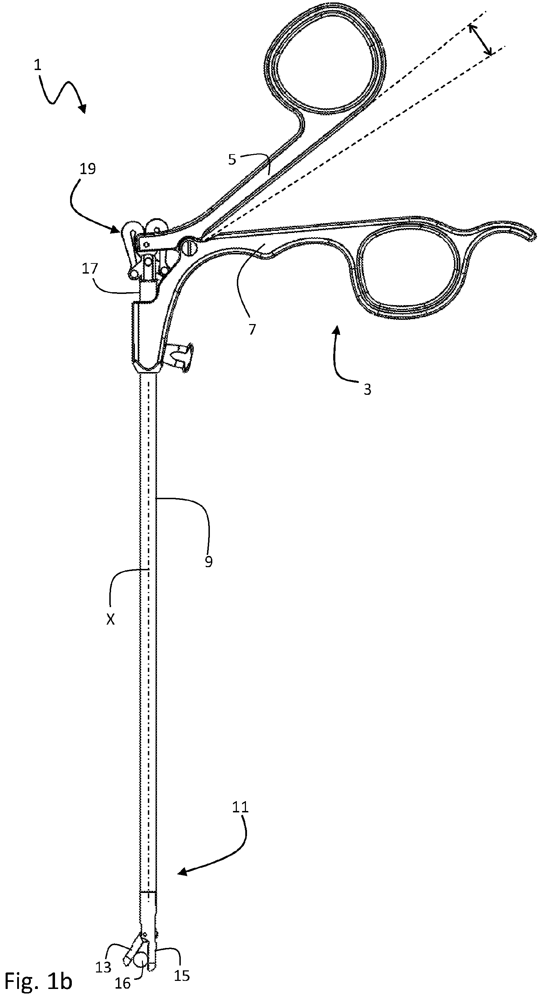

In Fig. 1a,b ist ein chirurgisches Instrument 1 in Form einer laparoskopischen Zange gezeigt, die in Fig. 1a fast geschlossen ist und in Fig. 1b weit geöffnet ist . Das Instrument weist eine manuell greifbare und betätigbare Betätigungseinrichtung 3 mit zwei Griffteilen 5, 7 auf, die wie eine Schere mit einer Hand gegriffen werden können. Ein erstes Griffteil 5 der zwei Griffteile 5, 7 kann mit dem Daumen des Anwenders relativ zu einem zweiten unbeweglichen Griffteil 7 der zwei Griffteile 5, 7 bewegt werden. Das erste Griffteil 5 ist dazu am zweiten Griffteil 7 verschwenkbar gelagert. Vom unbeweglichen zweiten Griffteil 7 erstreckt sich distalwärts entlang einer Längsachse X des chirurgischen Instruments 1 ein Schaft 9 zu einem Werkzeugkopf 11. Der Werkzeugkopf 11 ist in diesem Ausführungsbeispiel ein Zangenkopf mit zwei Werkzeugteilen 13, 15, die hier als Zangenmaulteile ausgebildet sind. Zumindest ein erstes 13 der zwei Werkzeugteile 13, 15 ist verschwenkbar gegenüber dem zweiten 15 der zwei Werkzeugteile 13, 15 gelagert. Es können ggf. beide Werkzeugteile 13, 15 relativ zum Schaft 9 verschwenkbar gelagert sein. In Fig. 1b ist ein relativ großes Gewebestück 16 zwischen den zwei Werkzeugteilen 13, 15 gezeigt. In Fig. 1a ist das Gewebestück 16 zu klein, um es sehen zu können.

Im Schaft 9 verläuft ein axial entlang einer Längsachse X beweglich im Schaft 9 verschiebbares Übertragungselement 17 in Form einer Zug-/Schubstange zur Übertragung einer manuellen Kraft von dem ersten Griffteil 5 auf das erste Werkzeugteil 13 und/oder zweite Werkzeugteil 15. Das Übertragungselement 17 ist proximalseitig mit dem ersten Griffteil 5 gelagert gekoppelt und distalseitig zumindest mit dem ersten Werkzeugteil 13 gekoppelt. Ein manuell bewirktes Schließen der Griffteile 5, 7 in Schließrichtung bewirkt daher eine distalwärtige Verschiebung des Übertragungselements 17 im Schaft 9, welche wiederum ein Schließen der Werkzeugteile 13, 15 bewirkt. Entsprechend bewirkt ein Öffnen der Griffteile 5, 7 in Öffnungsrichtung eine proximalwärtige Verschiebung des Übertragungselements 17 im Schaft 9, welche wiederum ein Öffnen der Werkzeugteile 13, 15 bewirkt. Die manuelle Kraft der Anwenderperson wird also auf den Werkzeugkopf 11 übertragen, um die Werkzeugteile 13, 15 gezielt bewegen zu können.

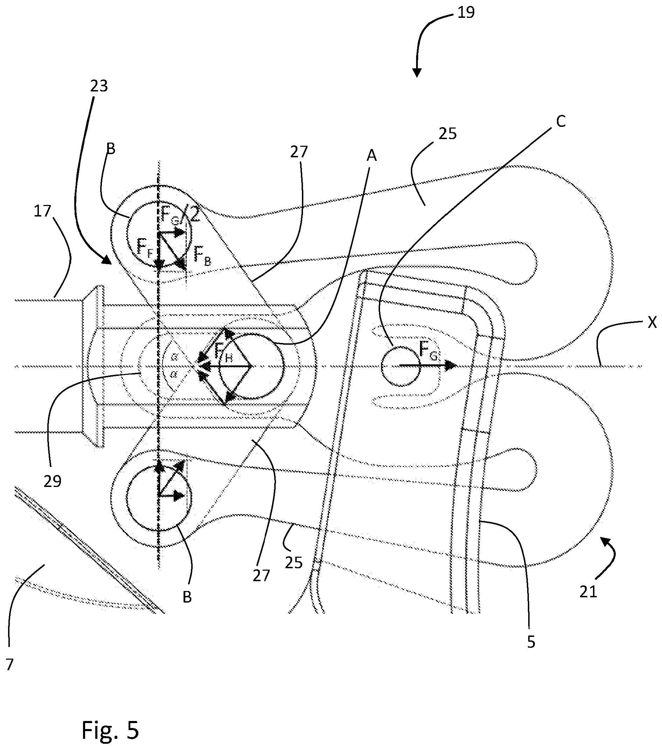

Um den Werkzeugkopf 11 vor einer Überlastung mit einer zu großen manuellen Kraft der Anwenderperson zu schützen, weist das chirurgische Instrument 1 eine Überlastungsschutzeinrichtung 19 auf mit einem Federelement 21 zur Begrenzung der in Schließrichtung auf das erste Werkzeugteil 13 übertragenen manuellen Kraft. Im Gegensatz zu aus dem Stand der Technik (SdT) bekannten Überlastungsschutzeinrichtungen, bei denen eine Feder zwischen Griffteil und Übertragungselement ab einer gewissen Kraft einfach gestaucht wird, weist die Überlastungsschutzeinrichtung 17 gemäß der vorliegenden Offenbarung einen Kniehebelmechanismus 23 auf, über welchen das Federelement 21 das Übertragungselement 17 in Schließrichtung, also proximalwärts zum ersten Griffteil 5 hin, mit einer Vorspannkraft F₀ vorspannt. Für die Vorspannkraft F₀ in Schließrichtung gilt: ${F}_{0} = {F}_{H} = 2 \frac{{F}_{F}}{tan {α}_{min}}$, wobei F_{F} eine radial nach innen wirkende Federkraft bei einem halben minimalen Öffnungswinkel αₘᵢₙ des Kniehebelmechanismus 23 ist (s. Fig. 5). Erst wenn eine über das erste Griffteil 5 auf das Federelement 21 ausgeübte proximalwärtige manuelle Zugkraft F_{G} größer als die Vorspannkraft F₀ ist, vergrößert sich der Öffnungswinkel 2α des Kniehebelmechanismus 23 und das Federelement 21 wird ausgelenkt.

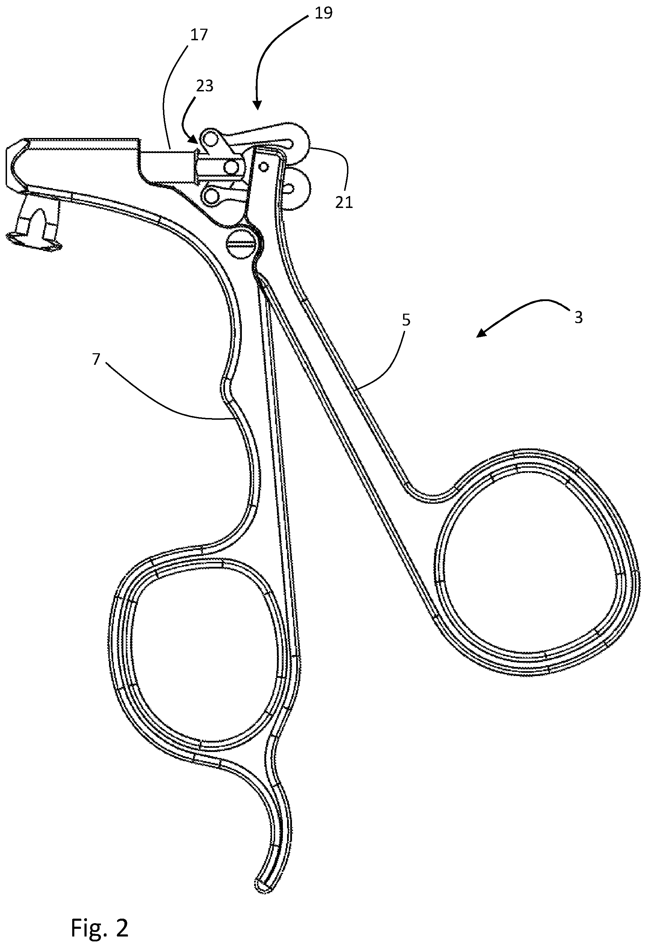

In Fig. 2 ist der proximale Teil des chirurgischen Instruments 1 mit der Betätigungseinrichtung 3 und der Überlastungsschutzeinrichtung 19 größer dargestellt, um die Überlastungsschutzeinrichtung 17 genauer zu zeigen. Wird das erste Griffteil 5 in Schließrichtung zum zweiten Griffteil 7 hin gedrückt, bewegt sich ein proximaler Lagerpunkt C proximalwärts. Das Federelement 21 ist am proximalen Lagerpunkt C dem ersten Griffteil 5 gelenkig verbunden. Dadurch wird das Federelement 21 vom Griffteil 5 in Schließrichtung proximalwärts gezogen. Das Federelement 21 ist symmetrisch M-förmig geschwungen und weist zwei Federbügel 25 auf, die sich auf gegenüberliegenden Seiten lateral von der Längsachse X vom proximalen Lagerpunkt C distalwärts erstrecken. Am distalen Ende der Federbügel 25 ist jeweils ein distaler Lagerpunkt B angeordnet.

Der Kniehebelmechanismus 23 ist zwischen den distalen Lagerpunkten B angeordnet und weist zwei gleich lange in einem Kniepunkt A miteinander gelenkig verbundene Kniehebelbeine 27 auf. Der Kniepunkt A liegt wie der proximalen Lagerpunkt C im Wesentlichen auf der Längsachse X, proximal von den distalen Lagerpunkten B, sodass der Kniehebelmechanismus 23 einen distalwärts geöffneten Öffnungswinkel 2α hat. Der Kniepunkt A ist ein Lagerpunkt am proximalen Ende des Übertragungselements 17, sodass sich das Übertragungselement 17 nur bewegt, wenn sich der Kniepunkt A bewegt.

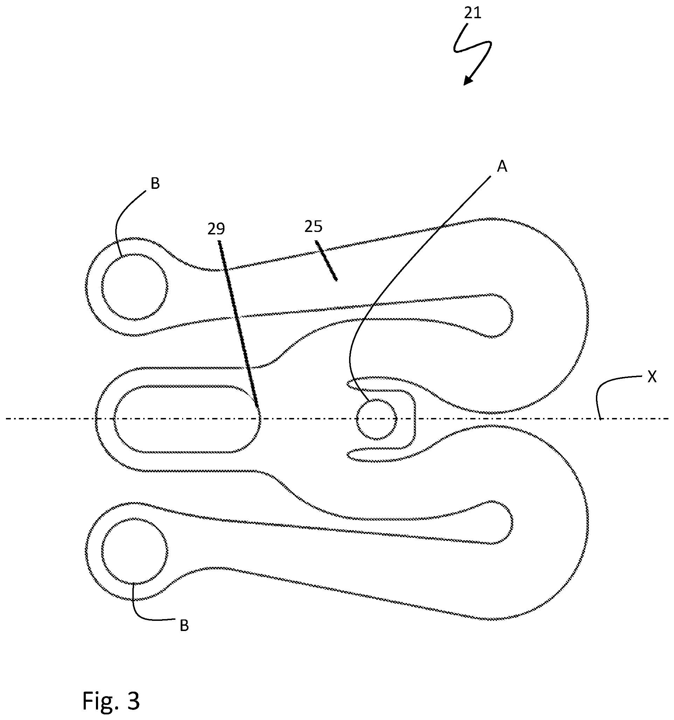

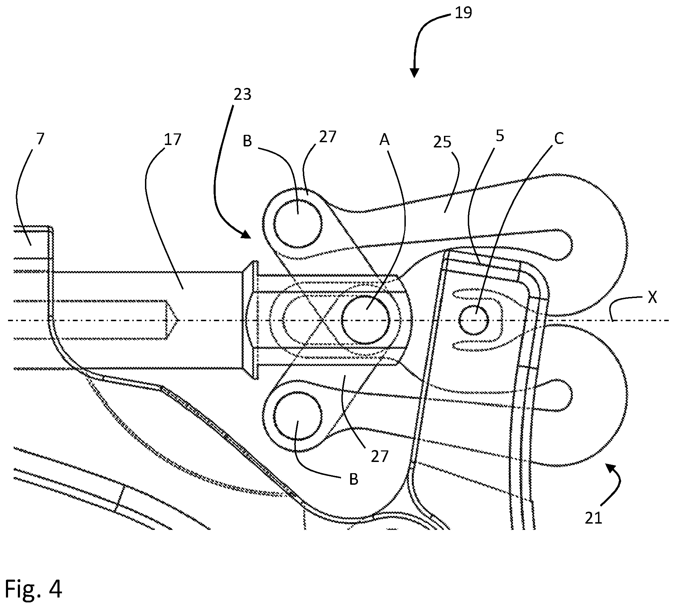

Zur geführten Bewegungsfreiheit des Kniepunkts A relativ zum Federelement 21 weist das Federelement 21 eine Führungsöffnung 29 in Form eines sich entlang der Längsachse X erstreckenden Langlochs auf, in welchem der Kniepunkt A gelagert ist. Das Langloch 29 ist besser in Fig. 3-6a zu erkennen. Solange kein Gewebe 16 zwischen den Werkzeugteilen 13, 15 Widerstand gegen das Schließen bildet, zieht das Federelement 21 den Kniepunkt A über den Kniehebelmechanismus 23 proximalwärts in Schließrichtung, ohne dass sich der Kniepunkt A in der Führungsöffnung 29 distalwärts bewegt. Die Reibungskräfte im chirurgischen Instruments 1 zur Bewegung der Werkzeugteile 13, 15 sollten so gering sein, dass die Überlastungsschutzeinrichtung 19 nicht aktiviert wird, solange kein Gewebe 16 zwischen den Werkzeugteilen 13, 15 Widerstand gegen das Schließen bildet.

Der Kniepunkt A ist in der proximalen Position innerhalb der Führungsöffnung 29 durch das Federelement 21 vorgespannt. Das heißt, die Länge L der Kniehebelbeine 27 und die Position der Führungsöffnung 29 ist so gewählt, dass die Federbügel 25 bereits etwas ausgelenkt sind und unter einer radial nach innen wirkenden Vorspannkraft F₀ stehen, wenn sich der Kniepunkt A noch wie gezeigt am proximalen Ende innerhalb der Führungsöffnung 29 befindet. Um sich innerhalb der Führungsöffnung 29 distalwärts zu bewegen, muss diese Vorspannkraft F₀ überwunden werden und sich der Öffnungswinkel 2α des Kniehebelmechanismus 23 gegen die Federkraft des Federelement 21 weiten. Dabei werden die distalen Lagerpunkte B gegen die Federkraft des Federelement 21 radial nach außen gedrückt.

Es ist wichtig zu verstehen, dass einerseits der Bewegungsspielraum des ersten Griffteils 5 begrenzt ist und anderseits die Überlastungsschutzeinrichtung 19 erst aktiviert wird, wenn Gewebe 16 zwischen den Werkzeugteilen 13, 15 Widerstand gegen das Schließen bildet. Ein sehr kleines Gewebestück 16 zwischen den Werkzeugteilen 13, 15 kann wegen der Vorspannkraft F₀ in der Überlastungsschutzeinrichtung 19 mit einer gewünschten Mindestkraft gehalten, gekniffen oder geschnitten werden, obwohl nur wenig Bewegungsspielraum für das erste Griffteil 5 verbleibt. Für größere Öffnungswinkel 2α des Kniehebelmechanismus 23 nimmt die auf Übertragungselement 17 übertragene manuelle Kraft ab. Größere Öffnungswinkel 2α des Kniehebelmechanismus 23 kommen allerdings nur bei großen, harten Gewebestücken 16 vor, die in der Praxis kaum eine Rolle spielen. Außerdem können größere Gewebestücke 16 zumindest teilweise in einem mittleren oder proximalen Abschnitt der Werkzeugteile 13, 15 gepackt werden, wo aufgrund des kürzeren Hebels die Greif-, Kneif- oder Schneidkraft höher ist. Die vorliegende Offenbarung zielt darauf ab, den in der Praxis häufig relevanten Pinzettengriff von kleinen Gewebestücken 16 am äußersten distalen Ende der Werkzeugteile 13, 15 zu verbessern, ohne den Überlastungsschutz aufzugeben.

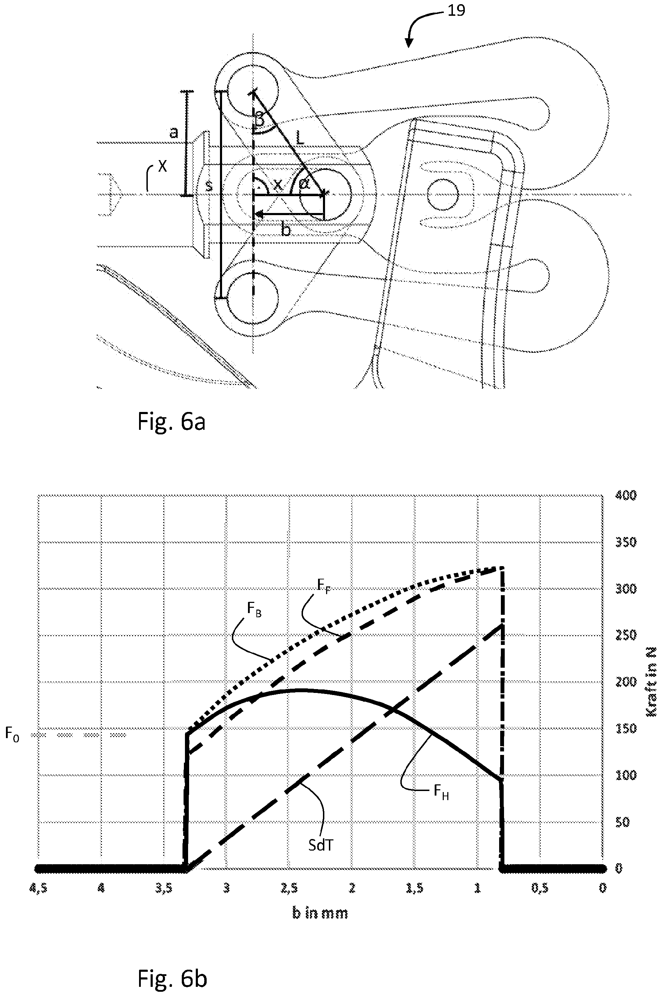

Fig. 5 verdeutlicht die in der Überlastungsschutzeinrichtung 19 herrschenden Kräfte. Wird eine manuelle Kraft F_{G} durch das erste Griffteil 5 in Schließrichtung ausgeübt, um das Federelement 21 am proximalen Lagerpunkt C proximalwärts zu ziehen, so teilt sich diese Kraft auf beiden distalen Lagerpunkte B auf, wo proximalwärts jeweils die halbe Kraft F_{G}/2 wirkt. Radial nach innen wirkt jeweils die Federkraft F_{F} des Federelements 21, sodass sich eine Zwischenkraft F_{B} entlang der Kniehebelbeine 27 auf den Kniepunkt ergibt. Sobald ein Gewebestück 16 zwischen den Werkzeugteilen 13, 15 einen ausreichenden Widerstand gegen das Schließen bildet, um das Federelement 21 auszulenken, wird auf das Übertragungselement 17 die Kraft ${F}_{H} = 2 {F}_{B} cos α = 2 {F}_{B} \frac{b}{L}$ übertragen, wobei b der momentane axiale Abstand zwischen den distalen Lagerpunkten B und dem Kniepunkt A ist (siehe Fig. 6a).

In Fig. 6b ist ein Kraftkurvendiagramm der Federkraft F_{F}, der Zwischenkraft F_{B} und der auf das Übertragungselement 17 übertragenen Kraft F_{H} im Vergleich zur übertragenen Kraft bei einer Feder aus dem Stand der Technik (SdT) als Funktion des momentanen axialen Abstands b zwischen den distalen Lagerpunkten B und dem Kniepunkt A gezeigt. Da sich der Abstand b im Verlauf der Aktivierung der Überlastungsschutzeinrichtung 19 verringert, nimmt b nach rechts hin im Diagramm ab. Befindet sich der Kniepunkt A am proximalen Ende der Führungsöffnung 29, beträgt der Abstand b zu Beginn der Aktivierung der Überlastungsschutzeinrichtung 19 in etwa 3,3 mm. Der entsprechend minimale Öffnungswinkel 2α des Kniehebelmechanismus 23 liegt bei ca. 120°, d.h. der halbe Öffnungswinkel α beträgt minimal ca. 60°.

Wenn im Falle eines großen, harten Gewebestücks 16 zwischen den Werkzeugteilen 13, 15 der Kniepunkt A bei maximaler Federauslenkung a am distalen Ende der Führungsöffnung 29 anschlägt, beträgt der Abstand b in etwa 0,8 mm. Die Führungsöffnung 29 erlaubt dem Kniepunkt A also einen maximalen Weg von 2,5 mm in der Führungsöffnung 29. Der Bewegungsspielraum des ersten Griffteils 5 kann entsprechend so eingeschränkt sein, dass der proximale Lagerpunkt C ebenfalls nur um 2,5 mm oder weniger axial beweglich ist. Damit kann das Federelement 21 unter Umgehung des Kniehebelmechanismus 23 in keinem Fall eine proximale Zugkraft direkt mit dem distalen Ende der Führungsöffnung 29 auf das Übertragungselement 17 einleiten. Am distalen Ende der Führungsöffnung 29 ist also auch das erste Griffteil 5 in der Schließstellung am Anschlag. Gleichzeitig ist in dieser Situation die Federauslenkung a sowie der Öffnungswinkel 2α des Kniehebelmechanismus 23 maximal bei ca. 166°, d.h. der halbe Öffnungswinkel α beträgt maximal ca. 83°. Die Länge L der Kniehebelbeine beträgt in diesem Beispiel etwa 6,6 mm.

Fig. 6b zeigt lang gestrichelt den Kraftverlauf der übertragenen Kraft bei einer Feder aus dem Stand der Technik (SdT) als Gerade, die ab Aktivierung der Überlastungsschutzeinrichtung stetig bis zur maximalen Federauslenkung ansteigt. Dagegen wird durch die überden Kniehebelmechanismus 23 bewirkte Vorspannung des Übertragungselements 17 in Schließrichtung mittels einer Vorspannkraft F₀ des Federelements 21 ein völlig anderer Verlauf, der auf das Übertragungselement 17 übertragenen Kraft F_{H} erzielt. Zwar nimmt die Federkraft F_{F} und auch die Zwischenkraft ${F}_{B} = \frac{{F}_{F}}{sin α}$ mit fallendem Abstand b bzw. steigendem Öffnungswinkel 2α des Kniehebelmechanismus 23 an, aber die auf das Übertragungselement 17 übertragene Kraft F_{H} steigt nur flach an und erreicht bei einem Abstand b von ca. 2,4 mm einen Höhepunkt und fällt für kleinere Abstände b. Dies liegt daran, dass der Kosinus-Term in *F_{H} =* 2*F_{B}* cos *α* im Winkelbereich von 60° bis 83° stark fällt. Die Länge L der Kniehebelbeine 25, die Federkonstante des Federelements 21 sowie der minimale Öffnungswinkel 2α des Kniehebelmechanismus 23, hier αₘᵢₙ = 60°, sind hier so gewählt, dass die auf das Übertragungselement 17 übertragene Kraft F_{H} unterhalb eines Abstands b von ca. 1,2 mm sogar unter das Niveau der Vorspannkraft F₀ fällt. Es sei angemerkt, dass im Kräftegleichgewicht immer *F_{G} = F_{H} =* 2*F_{B}* cos *α* gelten muss, also ${F}_{B} = \frac{{F}_{F}}{sin α} = \frac{{F}_{G}}{2 cos α}$.

Da die Kurvenbereiche mit kleinem Abstand b bzw. großen Öffnungswinkeln 2α des Kniehebelmechanismus 23 nur bei großen, harten Gewebestücken 16 zwischen den Werkzeugteilen 13, 15 vorkommen, ist der anfängliche Kurvenbereich mit großen Abständen b bzw. kleinen Öffnungswinkeln 2α des Kniehebelmechanismus 23 für die Praxis besonders relevant. Bei kleinen Gewebestücken 16 wird nämlich die Überlastungsschutzeinrichtung 19 auf einem Großteil des für den ersten Griffteil 5 zur Verfügung stehenden Weges in Schließrichtung nicht aktiviert. Wenn beispielsweise im Extremfall das Gewebestück 16 so klein ist, dass dem Griffteil 5 so wenig Platz bis zum Anschlag an das zweite Griffteil 7 verbleibt, dass es den proximalen Lagerpunkt C nur noch 1 mm proximalwärts ziehen kann, so ist die Kraftkurve nur Bereich des Abstands b zwischen 3,3 mm und 3,2 mm relevant. Bei einer aus dem Stand der Technik (SdT) einfachen Federstauchung als Überlastungsschutz könnte in Anfangsbereich kaum eine manuelle Kraft F_{H} übertragen werden (siehe lang gestrichelte Kurve).

Um die Kurve der auf das Übertragungselement 17 übertragenen Kraft F_{H} möglichst flach zu halten, ist die Länge L der Kniehebelbeine 25 im Rahmen anderweitiger konstruktiver Grenzen möglichst groß zu wählen. Die Vorspannkraft F₀ sollte bei möglichst geringer Federkonstante möglichst hoch sein, um einen möglichst kraftvollen Pinzettengriff kleiner Gewebestücke 16 zu erlauben. Ein hoher minimaler Öffnungswinkel 2α des Kniehebelmechanismus 23 ist vorteilhaft, um die maximale Federkraft F_{F} möglichst gering zu halten, um die strukturelle Belastung der Systemkomponenten möglichst niedrig zu halten.

### Bezugszeichenliste:

- 1: chirurgisches Instrument
- 3: Betätigungseinrichtung
- 5: erster Griffteil
- 7: zweiter Griffteil
- 9: Schaft
- 11: Werkzeugkopf
- 13: erstes Werkzeugteil
- 15: zweites Werkzeugteil
- 16: Gewebestück
- 17: Übertragungselement
- 19: Überlastungsschutzeinrichtung
- 21: Federelement
- 23: Kniehebelmechanismus
- 25: Federbügel
- 27: Kniehebelbeine
- 29: Führungsöffnung
- A: Kniepunkt
- B: distaler Lagerpunkt
- C: proximaler Lagerpunkt
- X: Längsachse
- L: Länge der Kniehebelbeine
- B: axialer Abstand zwischen Kniepunkt und distalem Lagerpunkt
- F_{H}: auf das Übertragungselement übertragene manuelle Kraft
- F_{G}: auf den proximalen Lagerpunkt wirkende manuelle Kraft
- F_{F}: Federkraft
- F_{B}: Zwischenkraft
- F₀: Vorspannkraft
- 2α: Öffnungswinkel des Kniehebelmechanismus

## Patentansprüche

1. Chirurgisches Instrument (1) zum chirurgischen Greifen, Kneifen, und/oder Schneiden von Gewebe innerhalb eines organischen Körpers, wobei das chirurgische Instrument (1) aufweist:
- eine Betätigungseinrichtung (3) mit zwei Griffteilen (5, 7), wobei mindestens ein erstes (5) der zwei Griffteile (5, 7) relativ zu einem zweiten (7) der zwei Griffteile (5, 7) translatorisch und/oder rotatorisch in eine Öffnungs- und Schließrichtung beweglich gelagert ist,
- einen Werkzeugkopf (11) mit zwei Werkzeugteilen (13, 15), wobei mindestens ein erstes (13) der zwei Werkzeugteile (13, 15) relativ zu einem zweiten (15) der zwei Werkzeugteile (13, 15) translatorisch und/oder rotatorisch in eine Öffnungs- und Schließrichtung beweglich gelagert ist,
- einen sich zwischen der Betätigungseinrichtung (3) und dem Werkzeugkopf (11) entlang einer Längsachse (X) erstreckenden Schaft (9), wobei ein proximales Ende des Schafts (9) mit der Betätigungseinrichtung (3) verbunden oder verbindbar ist, und wobei ein distales Ende des Schafts (9) mit dem Werkzeugkopf (11) verbunden oder verbindbar ist, wobei der Schaft (9) ein axial in einer Öffnungs- und Schließrichtung bewegliches Übertragungselement (17) zur Übertragung einer manuellen Kraft (F_{H}) von dem ersten Griffteil (5) auf das erste Werkzeugteil (13) aufweist, und
- eine Überlastungsschutzeinrichtung (19) mit einem Federelement (21) zur Begrenzung der in Schließrichtung auf das erste Werkzeugteil (13) übertragenen manuellen Kraft (F_{H}), wobei
die Überlastungsschutzeinrichtung (19) einen Kniehebelmechanismus (23) aufweist, über welchen das Federelement (21) das Übertragungselement (17) in Schließrichtung vorspannt, **dadurch gekennzeichnet, dass** der
Kniehebelmechanismus (23) zwei radial von der Längsachse (X) beabstandete distale Lagerpunkte (B) aufweist, über welche der Kniehebelmechanismus (23) jeweils mit einem von zwei Federbügeln (25) des Federelements (21) gelenkig verbunden ist.

2. Chirurgisches Instrument (1) nach Anspruch 1, wobei der Kniehebelmechanismus (23) in einem Kniepunkt (A) mit dem Übertragungselement (17) gelenkig verbunden ist.

3. Chirurgisches Instrument (1) nach Anspruch 2, wobei der Kniepunkt (A) proximal von den distalen Lagerpunkten (B) angeordnet ist.

4. Chirurgisches Instrument (1) nach einem der vorhergehenden Ansprüche, wobei das Federelement (21) einen proximalen Lagerpunkt (C) aufweist, über welchen das Federelement (21) mit dem ersten Griffteil (5) gelenkig verbunden ist.

5. Chirurgisches Instrument (1) nach Anspruch 2 und 4, wobei der proximale Lagerpunkt (C) proximal vom Kniepunkt (A) angeordnet ist.

6. Chirurgisches Instrument (1) nach einem der vorhergehenden Ansprüche, wobei das Federelement (21) derart mit dem ersten Griffteil (5) gekoppelt ist, dass es sich gegen die eigene Federspannung bei Bewegung des ersten Griffteils (5) in Schließrichtung unter Öffnung des Kniehebelmechanismus (23) radial nach außen aufspreizt, sobald die weitere Bewegung des ersten Werkzeugteils (13) in Schließrichtung durch Gewebe (16) blockiert wird.

7. Chirurgisches Instrument (1) nach einem der vorhergehenden Ansprüche, wobei das Federelement (21) zwei Federbügel (25) aufweist, die sich auf gegenüberliegenden Seiten lateral von der Längsachse (X) vom ersten Griffteil (5) zum Kniehebelmechanismus (23) erstrecken.

8. Chirurgisches Instrument (1) nach Anspruch 2, wobei das Federelement (21) eine sich entlang der Längsachse (X) erstreckende Führungsöffnung (29) aufweist, in welcher der Kniepunkt (A) geführt axial beweglich gelagert ist.

9. Chirurgisches Instrument (1) nach einem der vorhergehenden Ansprüche, wobei das Federelement (21) mit einer Vorspannkraft (F₀) vorgespannt ist, wenn der Kniehebelmechanismus (23) in einem minimalen Öffnungswinkel (2αₘᵢₙ) steht, wobei sich der Öffnungswinkel (2α) des Kniehebelmechanismus gegen die Vorspannkraft (F₀) weitet, sobald die weitere Bewegung des ersten Werkzeugteils (13) in Schließrichtung durch Gewebe (16) blockiert wird.

10. Chirurgisches Instrument (1) nach Anspruch 9, wobei die in Schließrichtung auf das erste Werkzeugteil (13) übertragene manuelle Kraft (F_{H}) in einem ersten Öffnungswinkelbereich des Kniehebelmechanismus (23) größer ist als die Vorspannkraft (F₀) und in einem oberhalb des ersten Öffnungswinkelbereichs liegenden zweiten Öffnungswinkelbereich des Kniehebelmechanismus (23) niedriger ist als die Vorspannkraft (F₀).

11. Chirurgisches Instrument (1) nach Anspruch 2, wobei der Kniehebelmechanismus (23) zwei gleich lange im Kniepunkt (A) miteinander gelenkig verbundene Kniehebelbeine (27) aufweist, wobei die Länge (L) der Kniehebelbeine (27) größer ist als ein axialer Bewegungsspielraum des Kniepunkts (A) relativ zu einem proximalen Lagerpunkt (C) des Federelements (21), über welchen das Federelement (21) mit dem ersten Griffelement (5) gelenkig verbunden ist.

12. Chirurgisches Instrument (1) nach einem der vorhergehenden Ansprüche, wobei der Werkzeugkopf (11) eine Zange, eine Schere und/oder eine Kneifzange bildet.

13. Chirurgisches Instrument (1) nach einem der vorhergehenden Ansprüche, wobei das zweite Griffelement (7) starr mit dem Schaft verbunden oder verbindbar ist.

14. Chirurgisches Instrument (1) nach einem der vorhergehenden Ansprüche, wobei das erste Griffelement (5) in einer Schließstellung anschlägt, wenn das erste Werkzeugteil (5) ohne blockierendes Gewebe (16) in einer Schließstellung bereits angeschlagen hat.

## Claims

1. A surgical instrument (1) for the surgical gripping, pinching and/or cutting of tissue within an organic body, wherein the surgical instrument (1) comprises:
- an actuation device (3) with two grip parts (5, 7), wherein at least a first (5) of the two grip parts (5, 7) is movably mounted relative to a second (7) of the two grip parts (5, 7) in a translatory and/or rotatory manner in an opening and closing direction,
- a tool head (11) with two tool parts (13, 15), wherein at least a first (13) of the two tool parts (13, 15) is movably mounted relative to a second (15) of the two tool parts (13, 15) in a translatory and/or rotatory manner in an opening and closing direction,
- a shank (9) which extends along a longitudinal axis (X) between the actuation device (3) and the tool head (11), wherein a proximal end of the shank (9) is connected or connectable to the actuation device (3), and wherein a distal end of the shank (9) is connected or connectable to the tool head (11), wherein the shank (9) comprises a transmission element (17) which is axially movable in an opening and closing direction for transmitting a manual force (F_{H}) from the first grip part (5) onto the first tool part (13), and
- an overload protection device (19) with a spring element (21) for limiting the manual force (F_{H}) which is transmitted onto the first tool part (13) in the closing direction,
wherein
the overload protection device (19) has a toggle lever mechanism (23), via which the spring element (21) biases the transmission element (17) in the closing direction,
**characterised in that** the
toggle lever mechanism (23) has two distal bearing points (B) which are radially distanced from the longitudinal axis (X) and via which the toggle lever mechanism (23) is articulately connected in each case to one of two spring bows (25) of the spring element (21).

2. The surgical instrument (1) according to claim 1, wherein the toggle lever mechanism (23) is articulately connected to the transmission element (17) in a toggle point (A).

3. The surgical instrument (1) according to claim 2, wherein the toggle point (A) is arranged proximally of the distal bearing points (B).

4. The surgical instrument (1) according to one of the preceding claims, wherein the spring element (21) has a proximal bearing point (C), via which the spring element (21) is articulately connected to the first grip part (5).

5. The surgical instrument (1) according to claim 2 and 4, wherein the proximal bearing point (C) is disposed proximally of the toggle point (A).

6. The surgical instrument (1) according to one of the preceding claims, wherein the spring element (21) is coupled to the first grip part (5) in such a manner that, upon movement of the first grip part (5) in the closing direction, the spring element spreads radially outwards counter to an intrinsic spring tension amid an opening of the toggle lever mechanism (23) as soon as the further movement of the first tool part (13) is blocked in the closing direction by tissue (16).

7. The surgical instrument (1) according to one of the preceding claims, wherein the spring element (21) has two spring bows (25) which on opposite sides extend laterally of the longitudinal axis (X) from the first grip part (5) to the toggle lever mechanism (23).

8. The surgical instrument (1) according to claim 2, wherein the spring element (21) has a guide opening (29) which extends along the longitudinal axis (X) and in which the toggle point (A) is mounted in an axially movably guided manner.

9. The surgical instrument (1) according to one of the preceding claims, wherein the spring element (21) is biased with a biasing force (F₀) when the toggle lever mechanism (23) is at a minimal opening angle (2aₘᵢₙ), wherein the opening angle (2a) of the toggle lever mechanism widens counter to the biasing force (F₀) as soon as the further movement of the first tool part (13) is blocked in the closing direction by way of tissue (16).

10. The surgical instrument (1) according to claim 9, wherein the manual force (F_{H}) which is transmitted onto the first tool part (13) in the closing direction is greater than the biasing force (F₀) in a first opening angle region of the toggle lever mechanism (23) and is lower than the biasing force (F₀) in a second opening angle region of the toggle lever mechanism (23) which lies above the first opening angle region.

11. The surgical instrument (1) according to claim 2, wherein the toggle lever mechanism (23) has two equally long toggle lever legs (27) which are articulately connected to one another in the toggle point (A), wherein the length (L) of the toggle lever legs (27) is larger than an axial range of motion of the toggle point (A) relative to a proximal bearing point (C) of the spring element (21), via which bearing point the spring element (21) is articulately connected to the first grip element (5).

12. The surgical instrument (1) according to one of the preceding claims, wherein the tool head (11) forms a forceps, a pair of scissors and/or a pinching forceps.

13. The surgical instrument (1) according to one of the preceding claims, wherein the second grip element (7) is rigidly connected or connectable to the shank.

14. The surgical instrument (1) according to one of the preceding claims, wherein the first grip element (5) abuts in a closure position when the first tool part (5) has already abutted in a closure position without tissue (16) which blocks.

## Revendications

1. Instrument chirurgical (1) pour saisir, pincer et/ou couper chirurgicalement des tissus à l'intérieur d'un corps organique, l'instrument chirurgical (1) présentant:
- un dispositif d'actionnement (3) à deux poignées (5, 7), au moins une première (5) des deux poignées (5, 7) par rapport à une seconde (7) des deux poignées (5, 7) s'appuyant de manière mobile en translation et/ou en rotation dans un sens d'ouverture et de fermeture,
- une tête d'outil (11) avec deux pièces d'outil (13, 15), au moins une première (13) des deux pièces d'outil (13, 15) s'appuyant de manière mobile en translation et/ou en rotation par rapport à une seconde (15) des deux pièces d'outil (13, 15) dans un sens d'ouverture et de fermeture,
- une tige (9) s'étendant entre le dispositif d'actionnement (3) et la tête d'outil (11) le long d'un axe longitudinal (X), une extrémité proximale de la tige (9) étant connectée ou reliée au dispositif d'actionnement (3), et une extrémité distale de la tige (9) étant reliée ou pouvant être reliée à la tête d'outil (11), la tige (9) présentant un élément de transmission mobile axialement dans un sens d'ouverture et de fermeture (17) pour transférer une force manuelle (F_{H}) de la première poignée (5) à la première pièce d'outil (13), et
- un dispositif de protection contre les surcharges (19) avec un élément à ressort (21) pour limiter la force manuelle (F_{H})
transmise dans le sens de fermeture à la première pièce d'outil (13), le dispositif de protection contre les surcharges (19) comportant un mécanisme de levier à genouillère (23) par lequel l'élément à ressort (21) précontraint l'élément de transmission (17) dans le sens de fermeture,
**caractérisé en ce que** le
mécanisme de levier à genouillère (23) présente deux points d'appui distaux (B) éloignés radialement de l'axe longitudinal (X), par lesquels le mécanisme de levier à genouillère (23) est relié de manière articulée respectivement à l'un des deux étriers à ressort (25) de l'élément à ressort (21).

2. Instrument chirurgical (1) selon la revendication 1, dans lequel le mécanisme de levier à genouillère (23) est relié de manière articulée à l'élément de transmission (17) à un point de genouillère (A).

3. Instrument chirurgical (1) selon la revendication 2, dans lequel le point de genouillère (A) est situé proximalement par rapport aux points d'appui distaux (B).

4. Instrument chirurgical (1) selon l'une des revendications précédentes, l'élément à ressort (21) présente un point d'appui proximal (C), par lequel l'élément à ressort (21) est relié de manière articulée à la première poignée (5).

5. Instrument chirurgical (1) selon les revendications 2 et 4, dans lequel le point d'appui proximal (C) est situé proximalement par rapport au genou (A).

6. Instrument chirurgical (1) selon l'une des revendications précédentes, dans lequel l'élément à ressort (21) est couplé à la première poignée (5) de telle sorte qu'il s'écarte radialement vers l'extérieur à l'encontre de sa propre tension de ressort lors du mouvement de la première poignée (5) dans le sens de fermeture, en ouvrant le mécanisme de levier à genouillère (23) dès que la poursuite du mouvement de la première pièce d'outil (13) est bloqué dans le sens de fermeture par les tissus (16).

7. Instrument chirurgical (1) selon l'une des revendications précédentes, dans lequel l'élément à ressort (21) présente deux étriers à ressort (25) qui s'étendent latéralement sur des faces opposées par rapport à l'axe longitudinal (X) de la première poignée (5) par rapport au mécanisme de levier à genouillère (23).

8. Instrument chirurgical (1) selon la revendication 2, dans lequel l'élément à ressort (21) présente une ouverture de guidage (29) qui s'étend le long de l'axe longitudinal (X) et dans laquelle le point de genouillère (A) est guidé axialement de manière mobile.

9. Instrument chirurgical (1) selon l'une des revendications précédentes, dans lequel l'élément à ressort (21) est précontraint avec une force de précontrainte (F₀) lorsque le mécanisme de levier à genouillère (23) se trouve dans un angle d'ouverture minimal (2aₘᵢₙ), l'angle d'ouverture (2a) du mécanisme de levier à genouillère s'élargissant à l'encontre de la force de précontrainte (F₀) dès que la poursuite du mouvement de la première pièce d'outil (13) est bloqué dans le sens de fermeture par les tissus (16).

10. Instrument chirurgical (1) selon la revendication 9, dans lequel la force manuelle (F_{H}) transférée dans le sens de fermeture à la première partie d'outil (13) est supérieure à la force de précontrainte (F₀) dans une première plage d'angle d'ouverture du mécanisme de levier à genouillère (23) et inférieure à la force de précontrainte (F₀) dans une seconde plage d'angle d'ouverture du mécanisme à genouillère (23) se trouvant au-dessus de la première plage d'ouverture.

11. Instrument chirurgical (1) selon la revendication 2, dans lequel le mécanisme de levier à genouillère (23) présente deux jambes de genouillère (27) reliées ensemble de manière articulée au niveau du point de genouillère (A), la longueur (L) des jambes de genouillère (27) étant supérieure à la marge de manœuvre axiale du point de genouillère (A) par rapport à un point d'appui proximal (C) de l'élément à ressort (21) par lequel l'élément à ressort (21) est relié de manière articulée à la première poignée (5).

12. Instrument chirurgical (1) selon l'une des revendications précédentes, dans lequel la tête d'outil (11) forme une pince, des ciseaux et/ou une tenaille.

13. Instrument chirurgical (1) selon l'une des revendications précédentes, dans lequel la seconde poignée (7) est reliée ou peut être reliée rigidement à la tige.

14. Instrument chirurgical (1) selon l'une des revendications précédentes, dans lequel la première poignée (5) bute à une position de fermeture lorsque la première pièce d'outil (5) a déjà buté sans tissu bloquant (16) dans une position de fermeture.
